(19) Europäisches Patentamt
European Patent Office
Office européen des brevets

(11) Veröffentlichungsnummer: **0 017 181**
**A1**

# (12) EUROPÄISCHE PATENTANMELDUNG

(21) Anmeldenummer: **80101657.7**

(22) Anmeldetag: **27.03.80**

(51) Int. Cl.³: **C 07 D 487/04**
C 07 D 233/64, C 07 D 403/06
C 07 C 123/00
//(C07D487/04, 243/00, 235/00)

(30) Priorität: **29.03.79 US 25219**
**22.10.79 US 86909**

(43) Veröffentlichungstag der Anmeldung:
**15.10.80 Patentblatt 80/21**

(84) Benannte Vertragsstaaten:
**AT BE CH DE FR GB IT LU NL SE**

(71) Anmelder: **F. HOFFMANN-LA ROCHE & CO. Aktiengesellschaft**
**CH-4002 Basel(CH)**

(72) Erfinder: **Field, George Francis**
**33 McKinley Avenue**
**West Caldwell, N.J., 07006(US)**

(72) Erfinder: **Zally, William J.**
**24 Milton Street**
**Cresskill, N.J. 07626(US)**

(74) Vertreter: **Lederer, Franz, Dr. et al.,**
**Patentanwälte Dr. Franz Lederer Reiner F. Meyer**
**Lucile-Grahn-Strasse 22**
**D-8000 München 80(DE)**

(54) **Neues Verfahren zur Herstellung von Imidazobenzodiazepinderivaten sowie Zwischenprodukte zu deren Herstellung.**

(57) Imidazobenzodiazepine der allgemeinen Formel

[Formel I: CH$_3$, N, N, N, X, Y — I]

worin X und Y Wasserstoff, Halogen oder Trifluormethyl bedeuten,
und pharmazeutisch akzeptable Säureadditionssalze davon werden nach einem neuen Verfahren hergestellt, gemäss welchem man eine Verbindung der allgemeinen Formel

[Formel II: CH$_3$, N, N, O, CH$_2$– N, C=O, O, X, Y — II]

worin X und Y obige Bedeutung haben, durch Behandeln mit einem niederen Alkylamin oder einem Hydrazin, oder durch saure oder basische Hydrolyse in eine entsprechende Verbindung der Formel I umwandelt, und erwünschtenfalls ein pharmazeutisch akzeptables Säureadditionssalz davon herstellt.

Die Verbindungen der Formel I sind bekannt und zeigen nützliche sedative, anxiolytische, muskelrelaxierende und antikonvulsive Wirkungen.

EP 0 017 181 A1

0017181
27. März 1980

F.Hoffmann-La Roche & Co.Aktiengesellschaft, Basel/Schweiz



RAN 4008/302

# Neues Verfahren zur Herstellung von Imidazobenzodiazepinderivaten sowie Zwischenprodukte zu deren Herstellung.

Die vorliegende Erfindung betrifft ein neues Verfahren zur Herstellung von Imidazobenzodiazepinen der allgemeinen Formel

I

worin X und Y Wasserstoff, Halogen oder Trifluormethyl bedeuten,

und pharmazeutisch akzeptable Säureadditionssalze davon.

Die in der vorliegenden Beschreibung benutzte Bezeichnung "Halo" oder "Halogen" bedeutet Fluor, Chlor, Brom oder Jod.

Nt/22.2.1980

Die Bezeichnung "niederes Alkyl" bedeutet geradkettige oder verzweigte, gesättigte Kohlenwasserstoffreste mit höchstens 7, vorzugsweise höchstens 4 Kohlenstoffatomen.

Die Bezeichnung "pharmazeutisch akzeptables Salz" bedeutet Salze mit anorganischen und organischen pharmazeutisch akzeptablen Säuren, wie Salzsäure, Bromwasserstoff, Salpetersäure, Schwefelsäure, Phosphorsäure, Zitronensäure, Ameisensäure, Maleinsäure, Essigsäure, Bernsteinsäure, Weinsäure, Methansulfonsäure, p-Toluolsulfonsäure und dergleichen. Solche Salze können von jedem Fachmann nach an sich bekannten Methoden leicht hergestellt werden.

Bevorzugt ist die Herstellung von 8-Chlor-6-(2-fluorphenyl)-1-methyl-4H-imidazo[1,5-a][1,4]benzodiazepin und dem Maleinsäuresalz davon.

Erfindungsgemäss können die Verbindungen der allgemeinen Formel I und ihre pharmazeutisch akzeptablen Säureadditionssalze dadurch hergestellt werden, dass man eine Verbindung der allgemeinen Formel

II

worin X und Y obige Bedeutung haben,
mit einem niederen Alkylamin oder einem Hydrazin umsetzt, oder einer sauren oder basischen Hydrolyse unterwirft und erwünschtenfalls eine so erhaltene Verbindung in ein pharmazeutisch akzeptables Säureadditionssalz überführt.

Die Verbindungen der Formel I sind bekannt und zeigen nützliche sedative, anxiolytische, muskelrelaxierende und antikonvulsive Wirkungen.

Die Umwandlung von Verbindungen der Formel II in Verbindungen der allgemeinen Formel I, sowie die Herstellung der Ausgangsstoffe der Formel II werden im folgenden Formelschema erläutert, worin die Symbole X und Y obige Bedeutung haben.

III → IV → V → VI → VII → VIII → II → I

$CH_3$ | $N{=}C{-}NH_2$ | NH | $CH_2$ | X | Y | III | IV

$CH_3$ | N | $CH_2OH$ | CHO | $CH_2$ | X | Y | VI | V

$CH_3$ | N·HCl | N | $CH_2Cl$ | $CH_2$ | O | X | Y | VII | VIII

$CH_3$ | N | C=O | $CH_2$ | O | X | Y | I | II

III ⟶ IV

Verbindungen der Formel III können nach einem Verfahren hergestellt werden, das Gassman et al. in Chemical Communications, 1973, p. 488 beschrieben hat. Die Herstellung der Desfluorverbindung der Formel III wird von Gall et al. in J. Med. Chem., 19, 1057 (1976) beschrieben.

Eine Verbindung der Formel III kann mit einer Lewis-Säure, wie Salzsäure, Zinkchlorid oder Aluminiumchlorid und Acetonitril umgesetzt werden. Geeignete Lösungsmittel für eine solche Reaktion sind inerte organische Lösungsmittel, wie Tetrahyrofuran, Methylenchlorid, Benzol, Toluol oder überschüssiges Acetonitril. Die Reaktionstemperatur kann von etwa Raumtemperatur bis etwa 150°C variiert werden, wobei die Siedetemperatur des gewählten Lösungsmittels bevorzugt ist.

IV ⟶ V

Verbindungen der Formel IV können anschliessend mit einem Halogenmalonaldehyd, wie Brommalonaldehyd in einem inerten organischen Lösungsmittel, wie ein $C_1$ bis $C_4$ Alkohol, z.B. Aethanol, Tetrahydrofuran oder Dimethylformamid umgesetzt werden. Die Reaktion kann in einem Temperaturbereich von etwa Raumtemperatur bis Siedetemperatur des gewählten Lösungsmittels durchgeführt werden, wobei die Siedetemperatur bevorzugt ist. Es können auch Puffersubstanzen, wie Triäthylammoniumacetat, zugegeben werden.

V ⟶ VI

Verbindungen der Formel V können mit einem Metallhydrid-Reduktionsmittel, wie Lithiumaluminiumhydrid oder Natriumborhydrid, in einem Lösungsmittel, wie in einem $C_1$ bis $C_4$ Alkohol, z.B. Aethanol, oder in einem tiefsiedenden Aether, wie Dioxan, umgesetzt werden. Die Reaktion kann bei Temperaturen von etwa 0° bis 80°C, vorzugsweise

bei Raumtemperatur, durchgeführt werden.

VI ⟶ VII

Verbindungen der Formel VI können anschliessend halogeniert werden mit einem Reagens, wie Thionylchlorid, Phosphoroxychlorid oder Phosphortrichlorid, in einem halogenierten Kohlenwasserstoff, wie Chloroform oder Methylenchlorid, oder in einem anderen inerten organischen Lösungsmittel, wie Benzol oder Toluol. Die Reaktion kann in einem Temperaturbereich von etwa 0° bis etwa 100°C, vorzugsweise in einem Bereich von 50-60°C, durchgeführt werden.

VII ⟶ VIII

Eine Verbindung der Formel VII kann anschliessend mit einer Base, wie einem Alkoxid, z.B. Natrium- oder Kaliummethoxid, Kalium-t-butoxid, oder einem Alkalimetallcarbonat, z.B. Natrium- oder Kaliumcarbonat, neutralisiert werden. Anschliessend gibt man ein Alkalimetallphthalimid, wie Kaliumphthalimid, hinzu. Die Reaktionstemperatur kann von etwa 0° bis 100°C variiert werden, wobei Raumtemperatur bevorzugt wird.

VIII ⟶ II

Eine Verbindung der Formel VIII kann mit Chromtrioxid oder Chromsäure in einem Essigsäure/Wasser-Gemisch umgesetzt werden, wobei man in einem Temperaturbereich von etwa 0° bis 120°C arbeiten kann. Vorzugsweise arbeitet man bei Siedetemperatur des verwendeten Lösungsmittelgemisches, z.B. 80-90°C.

II ⟶ I

Eine Verbindung der Formel II kann anschliessend mit einer wässrigen Lösung eines niederen Alkylamins, z.B. mit Methylamin, umgesetzt werden. Als Lösungsmittel kann man einen $C_1$ bis $C_4$ Alkohol verwenden, vorzugsweise Aethanol. Die Reaktion wird vorzugsweise bei Raumtemperatur durchgeführt. Das Endprodukt kann anschliessend durch bekannte Filtriertechniken isoliert werden.

Ein weiteres Verfahren, um Verbindung der allgemeinen Formel I zu erhalten, ist dadurch gekennzeichnet, dass man Verbindungen der Formel II mit Hydrazin in einem inerten Lösungsmittel, wie Aethanol, einer Mischung aus Aethanol und Chloroform, Tetrahydrofuran oder wässrigem Aethanol umsetzt. Die Reaktionstemperatur kann in einem Bereich von etwa Raumtemperatur bis etwa 100°C variiert werden, wobei man vorzugsweise bei Siedetemperatur des gewählten Lösungsmittels arbeitet. Das entstandene Produkt kann mit verdünnter Mineralsäure ausgeschüttelt werden und anschliessend durch Neutralisieren der sauren Lösung erhalten werden.

Eine dritte Methode, um Verbindungen der Formel I zu erhalten, besteht in der sauren oder basischen Hydrolyse von Verbindungen der Formel II. Für eine saure Hydrolyse kann man z.B. eine 30 prozentige Lösung einer Mineralsäure, wie Salzsäure, Bromwasserstoff, Schwefelsäure oder Phosphorsäure verwenden. Vorzugsweise arbeitet man bei Siedetemperatur oder wenig darunter. Für eine basische Hydrolyse können Alkalimetallhydroxide, wie Kalium- oder Natriumhydroxid verwendet werden. Inerte organische Lösungsmittel, wie sie oben aufgeführt sind, können als Lösungsvermittler zugegeben werden. Die Reaktion wird vorzugsweise bei Siedetemperatur des gewählten Lösungsmittels oder wenig darunter durchgeführt.

Die folgenden Beispiele sollen die vorliegende Erfindung erläutern, sie jedoch in keiner Weise einschränken. Alle Temperaturen sind in Celsius Graden angegeben.

Beispiel 1

2,5 l 2-Propanol werden unter Rühren mit 500 g (2 Mol) 2-Amino-5-chlor-2'-fluorbenzophenon versetzt. Man rührt die Mischung bis sich der Grossteil des Ausgangsmaterials gelöst hat, erwärmt auf etwa 60° und gibt 25 g (0,66 Mol) Natriumborhydrid hinzu. Der Ansatz wird unter Rühren während 1 Stunde zum Rückfluss erhitzt. Anschliessend lässt man langsam abkühlen und gibt 50 ml Eisessig hinzu, wobei ein gallertartiger Niederschlag entsteht. Die Mischung wird portionenweise mit 6 l Wasser verdünnt, wobei sich der gallertartige Niederschlag nach der ersten Zugabe auflöst. Die Zugabe wird fortgesetzt bis die Lösung trübe wird. Anschliessend wird gekratzt, angeimpft und gerührt bis ein körniger Niederschlag entsteht. Dann gibt man das restliche Wasser portionenweise hinzu, wobei man jeweils wartet bis der entstandene Niederschlag koaguliert hat. Nach der letzten Zugabe rührt man noch 1,5 Stunden, filtriert das Produkt ab und wäscht es mit Wasser und anschliessend mit 500 ml Hexan. Man lässt über Nacht an der Luft trocknen und erhält so 600-750 g rohen 2-Amino-5-chlor-α-(2-fluorphenyl)benzylalkohol, der direkt weiterverarbeitet wird. Eine Probe wird aus wässrigem Methanol umkristallisiert und ergibt reines Material vom Schmelzpunkt 94-98°.

Beispiel 2

2,5 l 6N Salzsäure werden auf 50-75° erwärmt und unter Rühren mit 300-340 g rohem 2-Amino-5-chlor-α-(2-fluorphenyl)benzylalkohol versetzt. Dabei kann ein dunkles Oel entstehen. Unter fortgesetztem Erwärmen und Rühren gibt man anschliessend 400-425 g Zinkstaub über einen Zeitraum von 15-20 Minuten hinzu und rührt während 1 Stunde unter Rückfluss. Anschliessend kühlt man in einem Eisbad unter Rühren auf etwa 5-10° ab, wobei ein körniger, gelber Niederschlag entsteht. Dieser Niederschlag wird

abfiltriert und zwischen 1,5 l Aether und 500 ml Wasser verteilt, wobei Spuren von Zink nicht stören. Die Mischung wird mit 300 ml konzentriertem Ammoniak basisch gestellt, wobei aus der wässrigen Phase ein weisser Niederschlag ausfällt, der sich wieder auflöst. Die wässrige Phase wird abgetrennt; die ätherische Phase wird mit 500 ml Wasser gewaschen, über Kaliumcarbonat getrocknet und im Vakuum eingedampft. Das so erhaltene orange Oel wird in 250 ml Toluol aufgenommen und wiederum eingedampft. Anschliessend löst man den Rückstand in 400-500 ml Aether und leitet durch diese Lösung Chlorwasserstoff hindurch, zuerst bei Raumtemperatur und dann unter Kühlen in einem Eisbad. Nach Sättigung mit Chlorwasserstoff lässt man die Mischung in einem Eisbad während 1 Stunde stehen und filtriert den entstandenen Niederschlag ab. Durch Umkristallisieren einer Probe aus Acetonitril erhält man 4-Chlor-2-(2-fluorbenzyl)anilin vom Schmelzpunkt 175-181°.

Beispiel 3

Eine Mischung aus 500 g (3,045 Mol) Malonaldehyd-bis-(dimethylacetal), 550 ml Wasser und 22 ml konzentrierter Salzsäure wird während 15 Minuten gerührt, bis eine klare Lösung entsteht. Anschliessend gibt man über einen Zeitraum von 20 Minuten 160 ml (3,11 Mol) Brom hinzu, wobei man die Reaktionstemperatur durch Kühlen mit einem Eisbad zwischen 20 und 30° hält. Nach beendeter Zugabe rührt man noch während 1 Stunde bei Raumtemperatur und engt anschliessend im Vakuum auf etwa 700 ml ein, wobei die Badtemperatur etwa 50-60° beträgt. Man lässt über Nacht im Kühlschrank stehen, filtriert den Niederschlag ab und wäscht ihn portionenweise mit 250 ml kaltem 50-prozentigem Methanol. Der Brommalonaldehyd wird in einem Vakuumofen getrocknet, wobei die Temperatur 60° nicht übersteigen soll.

Beispiel 4

Durch eine Suspension von 13,65 g (50 mMol) 4-Chlor-2-(2-fluorbenzyl)anilin in 200 ml Acetonitril wird unter Rühren Chlorwasserstoff hindurchgeleitet, wobei die Temperatur auf etwa 38° ansteigt. Anschliessend wird zum Sieden erhitzt, wobei der Chlorwasserstoffstrom weiterhin aufrechterhalten wird. Nach 1 Stunde wird die Mischung im Vakuum eingedampft, wobei ein blasser halbfester Niederschlag entsteht. Dieser wird zwischen 100 ml Methylenchlorid und etwa 30 ml 3N Natronlauge verteilt. Die wässrige Phase wird mit 100 ml Methylenchlorid ausgeschüttelt, und die vereinigten organischen Phasen werden mit 50 ml halbgesättigter Kochsalzlösung gewaschen, über Kaliumcarbonat getrocknet und im Vakuum eingedampft. Das so erhaltene hellgelbe Oel wird aus Hexan, das wenig Methylenchlorid enthält, kristallisiert und ergibt 4-Chlor-2-(2-fluorbenzyl)-N-(1-aminoäthyliden)anilin vom Schmelzpunkt 115-119°.

Beispiel 5

Eine gerührte Suspension von 98,7 g (0,357 Mol) 4-Chlor-2-(2-fluorbenzyl)-N-(1-aminoäthyliden)anilin in 500 ml 2-Propanol wird mit 55 g (0,364 Mol) Brommalonaldehyd, 250 ml 2-Propanol, 25 ml Eisessig und 59 ml Triäthylamin versetzt. Die Mischung wird während 1 Stunde unter Rückfluss gerührt, wobei eine orange Lösung entsteht. Man lässt über Nacht abkühlen und engt im Vakuum ein, wobei ein oranger Rückstand anfällt. Der Rückstand wird in 2 l Eiswasser und 100 ml Aether aufgenommen, der einige Impfkristalle enthält. Diese Mischung wird während etwa 2 Stunden gerührt, bis sich das Produkt verfestigt hat und der Grossteil des Aethers verdampft ist. Der 2-[Methyl-1-[4-chlor-2-(2-fluorbenzyl)phenyl]-1H-imidazol-5-carboxaldehyd wird abfiltriert und mit Wasser und Hexan gewaschen. Eine Probe wird aus Hexan umkristallisiert

und ergibt reines Material vom Schmelzpunkt 88-90°.

Beispiel 6

Eine auf 10° gekühlte Suspension von 140 g rohem 2-Methyl-1-[4-chlor-2-(2-fluorbenzyl)phenyl]-1H-imidazol-5-carboxaldehyd in 1 l Methanol wird über einen Zeitraum von 10 Minuten portionenweise mit 27,6 g (0,73 Mol) Natriumborhydrid versetzt, wobei die Temperatur auf etwa 15° ansteigt. Nach Rühren bei Raumtemperatur während 1 Stunde wird die Mischung mit Eisessig neutralisiert, im Vakuum eingedampft und unter Rühren und Kühlen mit Wasser verdünnt. Nach Rühren über Nacht wird abfiltriert, mit Wasser gewaschen und getrocknet. Man erhält 2-Methyl-1-[4-chlor-2-(2-fluorbenzyl)phenyl]-1H-imidazol-5-methanol vom Schmelzpunkt 152-155°.

Beispiel 7

Eine auf 10° gekühlte Lösung von 100 g (0,303 Mol) 2-Methyl-1-[4-chlor-2-(2-fluorbenzyl)phenyl]-1H-imidazol-5-methanol in 1 l Chloroform wird unter Rühren über einen Zeitraum von 15 Minuten mit 43,2 g (0,363 Mol) Thionylchlorid versetzt. Während der Zugabe wird die Temperatur der Reaktionsmischung durch Kühlen in einem Eis/Wasser-Bad zwischen 10° und 20° gehalten. Anschliessend rührt man noch während 2 Stunden bei Raumtemperatur. Die Lösung wird im Vakuum eingedampft, dabei erhält man einen gelben Rückstand. Man nimmt in 400 ml Methylenchlorid auf, filtriert, gibt 400 ml Aether hinzu und filtriert den entstandenen Niederschlag ab. Nach Trocknen im Vakuum erhält man 5-(Chlormethyl)-2-methyl-1-[4-chlor-2-(2-fluorbenzyl)phenyl]-1H-imidazol vom Schmelzpunkt 169-172° (Zersetzung).

Beispiel 8

Eine auf 10° gekühlte Suspension von 89,4 g (0,232 Mol) 5-(Chlormethyl)-2-methyl-1-[4-chlor-2-(2-fluorbenzyl)phenyl]-1H-imidazol in 900 ml Tetrahyrofuran wird mit 26,2 g (0,232 Mol) Kalium-t-butoxid versetzt. Die Temperatur der Reaktionsmischung wird dabei durch Kühlen mit einem Eisbad auf 20° gehalten. Anschliessend gibt man 51,5 g (0,278 Mol) Kaliumphthalimid hinzu und rührt während 2 Stunden unter Rückfluss. Man lässt auf Raumtemperatur abkühlen, giesst auf 1,2 l Eiswasser und schüttelt dreimal mit je 600 ml Methylenchlorid aus. Die vereinigten organischen Phasen werden über Natriumsulfat getrocknet und zur Trockene eingedampft. Das verbleibende Oel wird aus 180 ml Aether kristallisiert; dabei erhält man 2-Methyl-1-[4-chlor-2-(2-fluorbenzyl)phenyl]-5-phthalimidomethyl-1H-imidazol vom Schmelzpunkt 152-156°.

Beispiel 9

Eine Lösung von 53,1 g (0,116 Mol) 2-Methyl-1-[4-chlor-2-(2-fluorbenzyl)phenyl]-5-phthalimidomethyl-1H-imidazol in 600 ml Eisessig wird unter Rühren mit einer Chromsäurelösung (hergestellt aus 69,3 g (0,693 Mol) Chromtrioxid, 200 ml Wasser und 61,2 ml konzentrierter Schwefelsäure) versetzt. Anschliessend erwärmt man unter Rühren während 4 Stunden auf dem Dampfbad. Nach dem Abkühlen wird die überschüssige Chromsäure durch langsame Zugabe von 300 ml 2-Propanol zerstört, wobei die Temperatur 25° nicht übersteigen soll. Man neutralisiert unter Kühlen mit 3 l 6N Natronlauge und extrahiert sechsmal mit je 600 ml Methylenchlorid. Die vereinigten organischen Phasen werden zweimal mit je 750 ml Wasser und mit 750 ml halbgesättigter Kochsalzlösung gewaschen, über Magnesiumsulfat getrocknet und im Vakuum eingedampft. Der Rückstand wird aus Aether kristallisiert und ergibt 2-Methyl-1-[4-chlor-2-(2-fluorbenzoyl)phenyl]-5-phthalimidomethyl-1H-imidazol vom Schmelzpunkt 192-196°. Aus Essigester erhält man eine analytische Probe vom Schmelzpunkt 196-199°.

## Beispiel 10

Eine gerührte Suspension von 2-Methyl-1-[4-chlor-2-(2-fluorbenzoyl)phenyl]-5-phthalimidomethyl-1H-imidazol in 175 ml Aethanol wird mit 55,2 ml einer 40-prozentigen wässrigen Methylaminlösung versetzt. Die Mischung wird über Nacht bei Raumtemperatur gerührt, im Vakuum eingedampft und der Rückstand mit 350 ml Essigester behandelt. Der Festkörper wird abfiltriert, die Lösung wird im Vakuum eingedampft und der dabei erhaltene Rückstand wird noch einmal gleich behandelt. Das Filtrat wird im Vakuum eingedampft und ergibt ein Oel, das man in 75 ml heissem Aethanol auflöst. Dazu gibt man eine Lösung von 9,8 g (84,5 mMol) Maleinsäure in 10 ml heissem Aethanol, verdünnt die Lösung mit 175 ml Aether und kühlt in einem Eisbad ab. Dabei erhält man das Maleinsäuresalz von 8-Chlor-6-(2-fluorphenyl)-1-methyl-4H-imidazo[1,5-a][1,4]-benzodiazepin vom Schmelzpunkt 110-115°. Durch Umkristallisieren aus Aethanol/Aether erhält man reines Produkt vom Schmelzpunkt 114-117°.

## Patentanprüche

1. Verfahren zur Herstellung von Imidazobenzodiazepinen der allgemeinen Formel

I

worin X und Y Wasserstoff, Halogen oder Trifluormethyl bedeuten,

und pharmazeutisch akzeptablen Säureadditionssalzen davon, dadurch gekennzeichnet, dass man eine Verbindung der allgemeinen Formel

II

worin X und Y obige Bedeutung haben,

mit einem niederen Alkylamin oder einem Hydrazin umsetzt, oder einer sauren oder basischen Hydrolyse unterwirft und erwünschtenfalls eine so erhaltene Verbindung in ein pharmazeutisch akzeptables Säureadditionssalz überführt.

2. Verfahren gemäss Anspruch 1, dadurch gekennzeichnet, dass man eine Verbindung der allgemeinen Formel II mit einem niederen Alkylamin behandelt.

3. Verfahren gemäss Anspruch 2, dadurch gekennzeichnet, dass man eine wässrige Methylaminlösung verwendet.

4. Verfahren gemäss einem der Ansprüche 1-3, dadurch gekennzeichnet, dass man 2-Methyl-1-[4-chlor-2-(2-fluorbenzoyl)phenyl]-5-phthalimidomethyl-1H-imidazol als Ausgangsmaterial verwendet.

5. Eine Verbindung der allgemeinen Formel

II

worin X und Y Wasserstoff, Halogen oder Trifluormethyl bedeuten.

6. Eine Verbindung der allgemeinen Formel

VIII

worin X und Y Wasserstoff, Halogen oder Trifluormethyl bedeuten.

7. Eine Verbindung der allgemeinen Formel

CH3
N·HCl
N
CH2Cl
X
CH2
Y
VII

worin X und Y Wasserstoff, Halogen oder Trifluormethyl bedeuten.

8. Eine Verbindung der allgemeinen Formel

CH3
N
N
CH2OH
X
CH2
Y
VI

worin X und Y Wasserstoff, Halogen oder Trifluormethyl bedeuten.

9. Eine Verbindung der allgemeinen Formel

CH3
N
N
CHO
X
CH2
Y
V

worin X und Y Wasserstoff, Halogen oder Trifluormethyl bedeuten.

10. Eine Verbindung der allgemeinen Formel

CH3
N=C-NH2
X
CH2
Y
IV

worin X und Y Wasserstoff, Halogen oder Trifluormethyl bedeuten.

11. Eine Verbindung gemäss einem der Ansprüche 5-10, worin X Chlor und Y Fluor bedeuten.

***

Patentanprüche "für Oesterreich"

1. Verfahren zur Herstellung von Imidazobenzodiazepinen der allgemeinen Formel

I

worin X und Y Wasserstoff, Halogen oder Trifluormethyl bedeuten,

und pharmazeutisch akzeptablen Säureadditionssalzen davon, dadurch gekennzeichnet, dass man eine Verbindung der allgemeinen Formel

II

worin X und Y obige Bedeutung haben,

mit einem niederen Alkylamin oder einem Hydrazin umsetzt, oder einer sauren oder basischen Hydrolyse unterwirft und erwünschtenfalls eine so erhaltene Verbindung in ein pharmazeutisch akzeptables Säureadditionssalz überführt.

2. Verfahren gemäss Anspruch 1, dadurch gekennzeichnet, dass man eine Verbindung der allgemeinen Formel II mit einem niederen Alkylamin behandelt.

3. Verfahren gemäss Anspruch 2, dadurch gekennzeichnet, dass man eine wässrige Methylaminlösung verwendet.

4. Verfahren gemäss einem der Ansprüche 1-3, dadurch gekennzeichnet, dass man 2-Methyl-1-[4-chlor-2-(2-fluorbenzoyl)phenyl]-5-phthalimidomethyl-1H-imidazol als Ausgangsmaterial verwendet.

Europäisches Patentamt

# EUROPÄISCHER RECHERCHENBERICHT

Nummer der Anmeldung

EP 80 10 1657.7

| EINSCHLÄGIGE DOKUMENTE | | | KLASSIFIKATION DER ANMELDUNG (Int.Cl.3) |
|---|---|---|---|
| Kategorie | Kennzeichnung des Dokuments mit Angabe, soweit erforderlich, der maßgeblichen Teile | betrifft Anspruch | |
| X | US - A - 4 005 099 (UPJOHN)<br>* Spalte 1; Beispiel 4 *<br>-- | 1,2 | C 07 D 487/04<br>C 07 D 233/64<br>C 07 D 403/06<br>C 07 C 123/00<br>//(C 07 D 487/04, 243/00, 235/00) |
| | DE - A1 - 2 651 809 (TEIJIN)<br>* Anspruch 32; Seiten 30 bis 32 *<br>-- | 1,2 | |
| | GB - A - 1 475 460 (UPJOHN)<br>* Beispiele 15,16 *<br>-- | 1 | RECHERCHIERTE SACHGEBIETE (Int. Cl.3) |
| | US - A - 3 941 803 (UPJOHN)<br>* Beispiele 33, 34 *<br>---- | 1 | C 07 C 123/00<br>C 07 D 233/64<br>C 07 D 403/06<br>C 07 D 487/04 |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 04-07-1980 | FROELICH |